# EUROPEAN PATENT SPECIFICATION

(11) **EP 1 543 826 B1**
(45) Date of publication and mention of the grant of the patent: **03.09.2008**
(21) Application number: 04078366.4
(22) Date of filing: 10.12.2004
(51) Int. Cl.: A61K 9/08, A61K 31/136

(54) **Concentrated aqueous solution of ambroxol**
Konzentrierte wässrige Ambroxollösung
Solution aqueous concentree de ambroxol

(30) Priority: 16.12.2003 IT MI20032463
(43) Date of publication of application: 22.06.2005
(73) Proprietor: Advance Holdings Limited, Floriana, Malta (ML)
(72) Inventor: Pifferi, Giorgio, 20147 Milano (IT)
(74) Representative: Marchi, Massimo

(56) References cited:
- EP-A- 0 208 144
- EP-A- 0 240 907
- EP-A- 0 546 846
- EP-A- 1 437 134
- WO-A-01/05378
- US-A- 4 831 057

## Description

This invention relates to a concentrated aqueous solution of ambroxol.

Ambroxol [trans-4-(2-amino-3,5-dibromobenzylamino)cyclohexanol] is a well known mucolytic drug available in various compositions suitable for oral or parenteral administration. However, oral compositions are by far preferred.

The international patent application WO 01/05378 discloses a tablet to be sucked for the treatment of sore throat symptoms.

Subsequently, two articles have been published (Deutsche Apotheker Zeitung; 17, 2113, 2002; J. Fischer et al. Arzneim. Forsch.; 52, 256, 2002) relating to controlled studies versus placebo, randomised and statistically significant, which prove the effectiveness on sore throat symptoms of tablets containing 20 or 30 mg of ambroxol. However, the authors believe that liquid forms, such as mouth washes and sprays, do not allow ambroxol to exercise its effectiveness on sore throat symptoms due to its short time of persistence in the mouth.

Moreover, currently available aqueous liquid forms could not exercise a sufficient action because the concentration of ambroxol is rather low due to the poor solubility in water of ambroxol hydrochloride, which is about 3% (w/v).

Therefore, there is still a great need of an aqueous solution, free of unpleasant odour and flavour, containing high concentrations of ambroxol.

In an effort to achieve said result, the Applicant investigated several aqueous solutions of acid addition salts of ambroxol, such as ambroxol hydrochloride, containing stable non ionic surfactants that are well known in the pharmaceutical field and frequently used also in the preparation of injectable aqueous compositions, such as alcohols and aliphatic acids of the families commercially known under the trade names Cremophor™ and Solutol™. However, it was not possible to obtain aqueous solutions of ambroxol hydrochloride at a concentration substantially higher than 3% (w/v).

During said tests, instead, it has been surprisingly found that an increase of the pH value from 4.5-5 to pH 6-6.5, or more, enables to obtain solutions wherein the concentration of ambroxol higher than 3% (w/v). Generally, such solutions contain of from 4% to 7% (w/v).

It is therefore an object of this invention to provide an aqueous solution comprising an acid addition salt of ambroxol and a surfactant, wherein said surfactant is an alcohol or an etoxylate aliphatic acid, the pH value is of from 6 to 7 and the ambroxol content is of from 4 to 7% (w/v) .

Typically, the acid addition salt of ambroxol is hydrochloride.

In general, the aqueous solution of this invention may comprise further pharmacologically active ingredients whose concurrent administration is useful provided they do not to interfere with the solubility of the acid addition salt of ambroxol in the solution of this invention.

Preferably, however, an acid addition salt of ambroxol is the sole pharmacologically active ingredient in the aqueous solution of this invention.

Advantageously, a pH value of from 6 to 7 is obtained by adding an inorganic or organic base, such as sodium or potassium hydroxide, sodium or potassium bicarbonate, triethanolamine, Tris [tris(hydroxy methyl)aminomethane], N-methylglucosamine, lysine, arginine and the like.

Preferably, the surfactant amount in the solution of this invention is of from 5 to 20% (w/v). Even more preferably, of from 8% to 13% (w/v).

Advantageously, the surfactant is Solutol™ 15 (Macrogol 15 hydroxystearate, Pharmacopea Europea IV Ed.).

An additional advantage of the solution of this invention is that it is stable for a long period of time. In a dark bottle at 25°C it is stable for at least 12 months.

As is well known, ambroxol is characterised by a particularly bitter aftertaste which is increasingly difficult to mask as concentration increases. Therefore, it is particularly difficult to mask the aftertaste of ambroxol even when the excipients present in the solution of this invention do not have an unpleasant taste.

The Applicant has surprisingly found that ammonium salts of glycyrrhizic acid are particularly suitable to mask the bitter aftertaste of ambroxol. The preferred salt is ammonium monoglycyrrhizinate.

For this purpose, the combined action of ammonium monoglycyrrhizinate with menthol has been found to be particularly effective.

The preferred ammonium monoglycyrrhizinate:menthol weight ratio is of from 0.5:10 to 5:10.

In turn, the preferred ammonium monoglycyrrhizinate:ambroxol weight ratio is of from 0.001:1 to 0.1:1. More preferably, said weight ratio is of from 0.01:1 to 0.05:1, even more preferably, it is of from 0.011:1 to 0.025:1.

The solution of this invention is suitable for mucolytic therapy in place of known ambroxol-based compositions. However, broad investigations have shown that, surprisingly, the solution of this invention is effective in the treatment of sore throat symptoms when administered in the form of sprays or mouth washes.

Preferred dosage forms of the solution of this invention are, therefore, sprays and mouthwashes.

The following examples further illustrates the invention, without limiting it.

### Example 1

A solution according to this invention comprises:

| Ingredient | %(w/v) |
|---|---|
| Ambroxol hydrochloride | 5.00 |
| Tris | 0.36 |
| Solutol HS 15 | 10.00 |
| Glycerol | 2.90 |
| Sodium Benzoate | 0.50 |
| Acesulphame | 1.50 |
| Xylitol | 20.00 |
| Ammonium monoglycyrrhizinate* | 0.0625 |
| Menthol** | 0.5 |
| Purified water up to | 100 ml |

| | |
|---|---|
| ^{*)} in 1.25 ml of a hydro-alcoholic solution; | |
| ^{**)} in 1 ml of alcoholic solution. | |

### Test 1

### Activity on sore throat symptoms

A double blind trial was conducted on a group of 30 patients with acute non complicated sore throat who were divided for randomisation purposes into three groups of 10 patients each.

The first group was treated with placebo, the second one was treated with the solution of Example 1 in the form of a mouthwash, the third one was treated with the solution of Example 1 in the form of a spray.

At the time of the evaluation, patients were asked to express pain on a scale from 0 (= no pain) to 5 (= highly intense pain).

The evaluation was conducted before treatment and 30, 60, 120 and 180 minutes after treatment.

In the case of mouthwash, the treatment consisted in rinsing the mouth with the solution under evaluation for one minute.

In the case of spray, the treatment consisted in spraying 0.20 ml of the solution under evaluation.

Results (pain intensity vs. placebo):
Mouthwash: 30 minutes (50%), 60 minutes (35%), 120 minutes (30%), 180 minutes (30%);
Spray: 30 minutes (47%), 60 minutes (34%), 120 minutes (28%), 180 minutes (27%).

## Claims

1. An aqueous solution, comprising an acid addition salt of ambroxol and a surfactant, wherein the surfactant is an alcohol or an etoxylate aliphatic acid, the pH value is of from 6 to 7 and the ambroxol content is of 4 to 7% (w/v).

2. An aqueous solution according to claim 1, wherein the acid addition salt of ambroxol is hydrochloride.

3. An aqueous solution according to claim 1, wherein the acid addition salt of ambroxol is the sole pharmacologically active ingredient.

4. An aqueous solution according to any one of the previous claims, wherein the amount of surfactant is of from 5 to 20% (w/v).

5. An aqueous solution according to claim 4, wherein the amount of surfactant is of from 8 to 13% (w/v).

6. An aqueous solution according to any one of the previous claims, wherein the surfactant is Solutol^{™} 15 (Macrogol 15 Hydroxystearate).

7. An aqueous solution according to any one of the previous claims, which further comprises an ammonium salt of glycyrrhizic acid.

8. An aqueous solution according to any one of the previous claims, which further comprises menthol.

9. An aqueous solution according to claim 7, wherein the ammonium salt of glycyrrhizic acid is ammonium monoglycyrrhizinate.

10. An aqueous solution according to claim 9, wherein the ammonium monoglycyrrhizinate:menthol weight ratio ranges from 0.5:10 to 5:10.

11. An aqueous solution according to claim 9, wherein the ammonium monoglycyrrhizinate:ambroxol weight ratio ranges from 0.001:1 to 0.1:1.

12. An aqueous solution according to claim 11, wherein the ammonium monoglycyrrhizinate:ambroxol weight ratio ranges from 0.01:1 to 0.05:1.

13. An aqueous solution according to claim 12, wherein the ammonium monoglycyrrhizinate:ambroxol weight ratio ranges from 0.011:1 to 0.25:1.

14. An aqueous solution as claimed in any one of the previous claims, wherein said solution is in spray or mouthwash form.

## Patentansprüche

1. Wässrige Lösung aus einem Säureadditionssalz von Ambroxol und einem Tensid, wobei das Tensid ein Alkohol oder eine Ethoxylat-Aliphatik-Säure ist, deren pH-Wert 6 bis 7 und dessen Ambroxolgehalt 4 bis 7% (w/v) beträgt.

2. Wässrige Lösung nach Anspruch 1, bei der das Säureadditionssalz von Ambroxol Hydrochlorid ist.

3. Wässrige Lösung nach Anspruch 1, bei der das Säureadditionssalz von Ambroxol der einzige arzneilich wirksame Bestandteil ist.

4. Wässrige Lösung nach einem der vorangegangenen Anspruch, bei der die Menge des Tensids 5 bis 20% (w/v) beträgt.

5. Wässrige Lösung nach einem der vorangegangenen Ansprüche, bei der die Menge des Tensids 8 bis 13% (w/v) beträgt.

6. Wässrige Lösung nach einem der vorangegangenen Ansprüche, bei der das Tensid Solutol^{™} 15 (Macrogol 15 Hydroxystearat) ist.

7. Wässrige Lösung nach einem der vorangegangenen Ansprüche, die weiterhin ein Ammoniumsalz von Glycyrrhizinsäure umfasst.

8. Wässrige Lösung nach einem der vorangegangenen Ansprüche, die weiterhin Menthol umfasst.

9. Wässrige Lösung nach Anspruch 7, bei der das Ammoniumsalz der Glycyrrhizinsäure Ammoniummonoglycyrrhizinat ist.

10. Wässrige Lösung nach Anspruch 9, bei der das Gewichtsverhältnis von Ammoniummonoglycyrrhizinat zu Menthol von 0,5:10 bis 5:10 reicht.

11. Wässrige Lösung nach Anspruch 9, bei der das Gewichtsverhältnis von Ammoniummonoglycyrrhizinat zu Ambroxol von 0,001:1 bis 0,1:1 reicht.

12. Wässrige Lösung nach Anspruch 11, bei der das Gewichtsverhältnis von Ammoniummonoglycyrrhizinat zu Ambroxol von 0,01:1 bis 0,05:1 reicht.

13. Wässrige Lösung nach Anspruch 12, bei der das Gewichtsverhältnis von Ammoniummonoglycyrrhizinat zu Ambroxol von 0,011:1 bis 0,25:1 reicht.

14. Wässrige Lösung nach einem der vorangegangenen Ansprüche, wobei die Lösung in Form eines Sprays oder einer Mundspülung vorliegt.

## Revendications

1. Solution aqueuse comprenant un sel d'addition d'acide d'ambroxol et un agent tensioactif, dans laquelle l'agent tensioactif est un alcool ou un éthoxylat d'acide aliphatique, la valeur de pH va de 6 à 7 et la teneur en ambroxol va de 4 à 7 % (p/v).

2. Solution aqueuse selon la revendication 1, dans laquelle le sel d'addition d'acide d'ambroxol est un chlorhydrate.

3. Solution aqueuse selon la revendication 1, dans laquelle le sel d'addition d'acide d'ambroxol est l'ingrédient pharmacologiquement actif unique.

4. Solution aqueuse selon l'une quelconque des revendications précédentes, dans laquelle la quantité d'agent tensioactif va de 5 à 20 % (p/v).

5. Solution aqueuse selon la revendication 4, dans laquelle la quantité d'agent tensioactif va de 8 à 13 % (p/v).

6. Solution aqueuse selon l'une quelconque des revendications précédentes, dans laquelle l'agent tensioactif est le Solutol^{™} 15 (hydroxystéarate de Macrogol 15).

7. Solution aqueuse selon l'une quelconque des revendications précédentes, qui comprend en outre un sel d'ammonium d'acide glycyrrhizique.

8. Solution aqueuse selon l'une quelconque des revendications précédentes, qui comprend en outre du menthol.

9. Solution aqueuse selon la revendication 7, dans laquelle le sel d'ammonium d'acide glycyrrhizique est le monoglycyrrhizinate d'ammonium.

10. solution aqueuse selon la revendication 9, dans laquelle le rapport en poids de monoglycyrrhizinate : menthol va de 0,5 : 10 à 5 : 10.

11. Solution aqueuse selon la revendication 9, dans laquelle le rapport en poids de monoglycyrrhizinate : ambroxol va de 0,001 : 1 à 0,1 : 1.

12. Solution aqueuse selon la revendication 11, dans laquelle le rapport en poids de monoglycyrrhizinate : ambroxol va de 0,01 : 1 à 0,05 : 1.

13. Solution aqueuse selon la revendication 12, dans laquelle le rapport en poids de monoglycyrrhizinate : ambroxol va de 0,011 : 1 à 0,25 : 1.

14. Solution aqueuse selon l'une quelconque des revendications précédentes, dans laquelle ladite solution est sous la forme de pulvérisation ou de bain de bouche.
